# EUROPEAN PATENT SPECIFICATION

(11) **EP 0 235 942 B1**
(45) Date of publication and mention of the grant of the patent: **23.03.1994**
(21) Application number: 87300850.2
(22) Date of filing: 30.01.1987
(51) Int. Cl.: C07D 295/12, A61K 31/395

(54) **Alkylenediamine derivatives**
Alkylendiamin-Derivate
Dérivés d'alkylènediamines

(30) Priority: 01.02.1986 JP 20497/86; 01.02.1986 JP 20498/86
(43) Date of publication of application: 09.09.1987
(73) Proprietor: NIPPON CHEMIPHAR CO., LTD., Chiyoda-ku Tokyo101 (JP)
(72) Inventor: Masaki, Mitsuo, Chiba-shi Chiba (JP); Shinozaki, Haruhiko, Oomiya-shi Saitama (JP); Satoh, Masaru, Koshigaya-shi Saitama (JP); Mortioh, Naoya, Kuki-shi Saitama (JP); Hashimoto,Koichi, Bunkyo-ku Tokyo (JP); Kamishiro, Toshiro, Misato-shi Saitama (JP)
(74) Representative: Arthur, Bryan Edward

(56) References cited:
- EP-A- 0 177 245
- CHEMICAL ABSTRACTS, vol. 80, no. 1, 7th January 1974, pages 293-294, abstract no. 3360e, Columbus, Ohio, US; L. Sh. PIRDZHANOV et al.: "Isoquinoline derivatives. IX. N-Substituted 1-methyl-6,7-dimethoxy-1,2,3,4-tetrahydroisoquinolines and their cyclic analogs"
- CHEMICAL ABSTRACTS, vol. 78, no. 9, 5th March 1973, page 517, abstract no. 58359a, Columbus, Ohio, US; A.L. MNDZHOYAN et al.: "Synthesis of N'-alkyl, dialkyl-, and heterocyclic aminoethyl-N-ethylhomoveratrylamines"
- BULLETIN DE LA SOCIETE CHIMIQUE DE FRANCE, no. 6, 1966, pages 1838-1846; E. SZARVASI et al.: "Quelques nouvelles structures naphtaléniques à activité antispasmodique"

## Description

The present invention relates to novel alkylenediamine derivatives. The alkylenediamine derivatives are favorably employable as glutamate blockers.

It is generally accepted that glutamate serves as an excitatory transmitter in the Crustacea. Further, it is also considered that glutamate is a potent candidate of the excipitory transmitter even in the central nervous system of mammal.

There is known γ-methylester of glutamic acid as a glutamate blocker which is effective to inhibit the above-mentioned functions of glutamate. However, the glutamate blocking function of the γ-methylester of glutamic acid is observed only when it is employed at high concentrations such as 10⁻²M to 10⁻³M. Accordingly, the γ-methylester in not satisfactory from the viewpoint of practical employment as the glutamate blocker.

It is further reported that Diltiazem and Caroberine also show inhibition against the functions of glutamate :see "Seitai no Kagaku" in Japanese language, 30(2); 82-91, 1979. However, the inhibitory function of these compounds are weak, as compared with other blockers employed in other transmission systems, such as anticholinergic agents against acetylcoline and antihistamines against histamine. For instance, at dose of 2 x 10⁻⁴M. Diltiazem and Caroberine both are effective only such low level as to inhibit approx. 30% of depolarization induced in the case of applying glutamic acid (1x10⁻⁴M) to the opener muscle of the first walking leg of the crayfish. Further, the action provided by these known compounds is not selective.

It has been reported in EP-A-177245 that aminoalcohol derivates such as 5-methyl-1-phenyl-2-(3-piperidinopropylamino) hexan-1-ol show the glutamate blocking function. However, the glutamate blocking functions of these aminoalcohol derivatives are not sufficiently high if the administration is made at a low level. It has also been reported in Bull. Soc. Chem. France, 6, (1966), 1838-1846, that certain amines with naphthyl, alkenyl and saturated nitrogen heterocyclic substituent groups and having anti-spasmodic activity have been prepared. However, their activity was less than that of papaverine.

An object of the present invention is to provide novel alkylenediamine derivatives which effectively function as glutamate blockers.

According to the present invention, there is provided an alkylenediamine derivative having either formula (I) or (II): 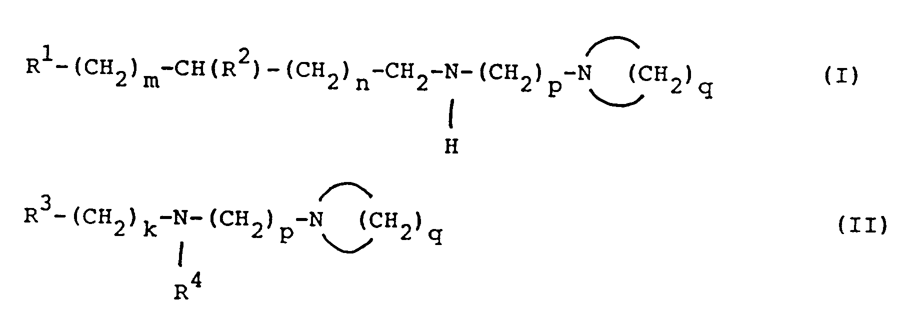
wherein
R¹ is a straight or branched chain alkyl group containing 3-8 carbon atoms, an alicyclic group containing 5-8 carbon atoms, a phenyl group, or an aralkyl group in which the alkyl moiety contains 1-4 carbon atoms; R² is a straight or branched chain alkyl group containing 4-8 carbon atoms, an alkoxy group containing 3-11 carbon atoms, an aliphatic hydrocarbon group containing an ester bonding and 3-11 carbon atoms, an aliphatic hydrocarbon group containing an ether bonding and 3-11 carbon atoms, or an aryloxy group; and each of m and n is an integer of 0 to 2, provided that m + n does not exceed 3; R³ is a straight or branched chain aliphatic hydrocarbon group containing 3-8 carbon atoms, an alicyclic group containing 5-8 carbon atoms, an aryl group, or an aralkyl group in which the alkyl moiety contains 1-4 carbon atoms; and k is an integer of 1 to 4; R⁴ is a straight or branched chain aliphatic hydrocarbon group containing 3-11 carbon atoms, an aliphatic hydrocarbon group containing an ester bonding and 3-11 carbon atoms, an aliphatic hydrocarbon group containing an ether bonding and 3-11 carbon atoms, or an aralkyl group in which the alkyl moiety contains an ether bonding and 2-5 carbon atoms;
p is an integer of 2 to 6;
and
q is an integer of 4 to 6.

It is to be noted that none of the specifically claimed compounds which follow accords exactly to the general formulae of Claim 1.

The alkylenediamine derivative of the invention can be prepared by a process which comprises reacting a carboxylic acid having the formula (V):

A - COOH (V)

wherein A is R¹-(CH₂)ₘ-CH(R²)-(CH₂)ₙ- or R³(CH₂)ₖ₋₁- or its reactive derivative with an amine derivative having the formula (VI): 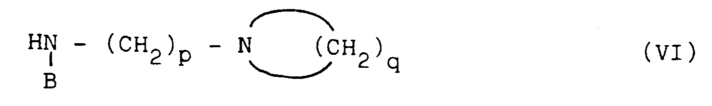
wherein B is H or R⁴ and each of p and q has the same meaning as above, to obtain a compound having the formula (VII): 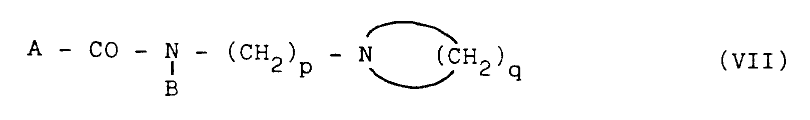
wherein each of A, B, p and q has the same meaning as above;
and reducing the compound of the formula (VII).

Alternatively, the alkylenediamine derivative of the invention can be prepared by a process which comprises reacting an amine compound having the formula (VIII): 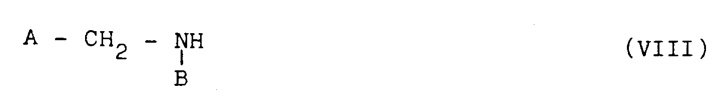
wherein each of A and B has the same meaning as above, or its reactive derivative with a haloganated compound having the formula (IX): 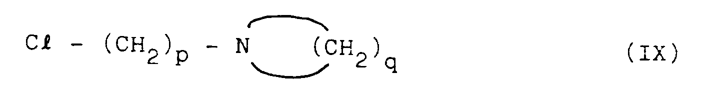
wherein each of p and q has the same meaning as above.

The alkylenediamine derivative of the present invention is particularly useful as glutamate blocker. The glutamate blocing action of the alkylendiamine derivative of the invention is prominently high as much as 10 to 100 times or more, as compared with the actions provided by the known glutamate blockers such as γ-methylester of glutamic acid, Diltiazem, and Caroberine.

It is known that convulsion is caused when glutamic acid is injected into brain of mammal. Accordingly, the alkylenediamine derivative of the present invention showing prominent glutamate blocking action is of value as pharmaceutical for neuriatria caused by unbalanced nervous system or abnormal exasperation of muscular pulse.

Further, the alkylenediamine derivative is of value as agricultural, chemical, particularly, as insecticide, because it is effective to block the transmission at neuromuscular junctions of insects in which glutamic acid serves as excitatory transmitter at their neuromuscular junctions, whereby decreasing actions of insects.

It has been further confirmed that the alkylenediamine derivative of the invention is low both in acute toxicity and subacute toxicity.

In the process of the present invention, the reaction of the compound of the formula (V) and the compound of the formula (VI) can be performed in the presence of a solvent. The reaction of the compound of the formula (VIII) and the compound of the formula (IX) also can be performed in the absence or presence of a solvent, and further can be performed in the presence of an alkali agent.

The above-mentioned reaction of the compound of the formula (VIII) and the compound of the formula (IX) is preferably performed at temperatures from room temperature to 160°C.

In the alkylenediamine derivative of the present invention, the above-mentioned R¹ preferably is a straight or branched chain alkyl group having 3-8 carbon atoms such as propoyl, isopropyl, butyl, isobutyl, pentyl, isopentyl, hexyl, heptyl, octyl, or 2-ethylhexyl, or phenyl.

The above-mentioned R² preferably is a straight or branced chain alkyl group having 4-8 carbon atoms such as butyl, isobutyl, pentyl, isopentyl, hexyl, heptyl, octyl, or 2-ethylhexyl. Otherwise, R² preferably is a straight or branched chain alkoxy group having 4-8 carbon atoms such as butoxy, isobutoxy, pentyloxy, isopentyloxy, hexyloxy, heptyloxy, octyloxy, or 2-ethylhexyloxy.

In the alkylenediamine derivative of the invention, each of "m", "n" preferably is 0 or 1, "k" preferably is 1 or 2 "p" preferably is 2 or 3, and "q" preferably is 5 or 6.

The alkylenediamine derivative of the invention is a compound wherein a heterocyclic group such as piperidinyl, pyrrolidinyl, or perhydroazepinyl is attached to a carbon atom of an alkylamine via the nitrogen of the hetero-ring. Therefore, the alkylenediamine can form a salt with an optionally selected organic or inorganic acid. Examples of the organic acids include oxalic acid, fumaric acid, maleic acid, citric acid, tartaric acid, p-toluenesulfonic acid, and methanesulfonic acid. Examples of the inorganic acids include hydrochlorica cid, sulfuric acid, nitric acid, hydrobromic acid, and phosphoric acid.

The alkylenediamine derivative of the invention can be employed in the form of a salt with any acid for the use as an insecticide, but ought to be in the form of a salt with a pharmaceutically acceptable acid. Examples of such acids include hydrochloric acid, fumaric acid, maleic acid, and methanesulfonic acid.

Representative examples of the alkylenediamine derivatives of the invention are given below whereby it is stressed that several of the examples are not comprised by claim 1;
1-[3-[4-methyl-1-(3-methylbutyl)pentylamino]propyl]-piperidine;
1-[3-[4-methyl-1-(3-methylbutyl)pentylamino]propyl]pyrrolidine;
1-[3-[4-methyl-1-(3-methylbutyl)pentylamino]propyl]perhydroazepine;
1-[3-[4-methyl-1-(3-methylbutyl)pentylamino]propyl]perhydroazocine
1-[2-[4-methyl-1-(3-methylbutyl)pentylamino]ethyl]piperidine;
1-[4-[4-methyl-1-(3-methylbutyl)pentylamino]butyl]piperidine;
1-[5-[4-methyl-1-(3-methylbutyl)pentylamino]pentyl]piperidine;
1-[3-(1-pentylhexylamino)propyl]piperidine;
1-[3-[4,4-dimethyl-1-(3,3-dimethylbutyl)pentylamino]propyl]piperidine;
1-[3-[2,3-dimethyl-1-(3-methylbutyl)pentylamino]propyl]piperidine;
1-[3-[1-(1-ethylpropyl)-4-methylpentylamino]propyl]piperidine;
1-[3-[4-methyl-1-(2-phenylethyl)pentylamino]propyl]piperidine;
1-[3-[4-methyl-1-(3-phenylpropyl)pentylamino]propyl]piperidine;
1-[3-[5-methyl-2-(2-phenylethyl)hexylamino]propyl]piperidine;
1-[3-(2-benzyl-5-methylhexylamino)propyl]piperidine;
1-[3-(3-benzyl-6-methylheptylamino)propyl]piperidine;
1-[3-(6-methyl-3-phenylheptylamino)propyl]piperidine;
1-[3-[1-(3-methylbutyl)hexylamino]propyl]piperidine;
1-[3-[4,4-dimethyl-1-(3-methylbutyl)pentylamino]propyl]piperidine;
1-[3-(1-benzyl-4-methylpentylamino)propyl]piperidine;
1-[3-(4-methyl-1-phenylpentylamino)propyl]piperidine;
1-[3-(5-methyl-2-phenylhexylamino]propyl]piperidine;
1-[3-(3-isopropoxy-1-phenylpropylamino)propyl]piperidine;
1-[3-[2-(3-methylbutyloxy)-2-phenylethylamino]propyl]piperidine;
1-[3-[2-phenoxy-2-phenylethylamino]propyl]piperidine;
3-phenyl-2-(3-piperidinopropylamino)propyl 3-methylbutanoate;
1-[3-(1-benzyl-4-methylpentylamino)propyl]pyrrolidine;
1-[3-(5-methyl-2-phenylhexylamino]propyl]pyrrolidine;
1-[3-(1-benzyl-4-methylpentylamino)propyl]perhydroazepine;
1-[2-(1-benzyl-4-methylpentylamino)ethyl]pyrrolidine;
1-[2-(1-benzyl-4-methylpentylamino)ethyl]piperidine;
1-[2-[4-methyl-1-(3-methylbutyl)pentylamino]ethyl]piperidine;
1-[3-[bis(3-methylbutyl)amino]propyl]piperidine;
1-[3-[N-(3-methylbutyl)-N-(4-methylpentyl)amino]propyl]piperidine;
1-[3-[N-(3-methylbutyl)-N-(5-methylhexyl)amino]propyl]piperidine;
1-[3-[N-hexyl-N-(3-methylbutyl)amino]propyl]piperidine;
1-[3-[N-heptyl-N-(3-methylbutyl)amino]propyl]piperidine;
1-[3-[N-(3-methylbutyl)-N-octylamino]propyl]piperidine;
1-[3-[N-(3-methylbutyl)-N-nonylamino]propyl]piperidine;
1-[3-[N-(3,3-dimethylbutyl)-N-(3-methylbutyl)amino]propyl]piperidine;
1-[4-[N-(3-methylbutyl)-N-(5-methylhexyl)amino]butyl]piperidine;
1-[2-[N-(3-methylbutyl)-N-(5-methylhexyl)amino]ethyl]piperidine;
1-[3-[N-(3-methylbutyl)-N-(5-methylhexyl)amino]propyl]pyrrolidine;
1-[3-[N-(3-methylbutyl)-N-(5-methylhexyl)amino]propyl]perhydroazepine;
1-[3-[N,N-bis(3,3-dimethylbutyl)amino]propyl]piperidine;
1-[3-[N-(2-benzyl-4-methylpentyl)-N-(3-methylbutyl)amino]propyl]piperidine;
The alkylenediamine derivative of the invention can be employed as a pharmaceutical in various forms of conventional pharmaceutical compositions such as powder, granules, tablet, injection composition, and suppository.

The alkylenediamine derivative of the invention can be employed as a pharmaceutical for neuriatria at dosage of 0.1 to 50 mg/day in an injection composition and 1 to 500 mg/day in an orally administerable composition. However, the dosage can be varied depending on the age, conditions, and the like of the patient.

The alkylenediamine derivative of the invention can be employed as an insecticide for exterminating harmful insects in the form of a simple aqueous solution or in combination with additives generally employed for the preparation of agricultural chemicals. Such compositions can be as such employed with no dilution or can be used with additional water when it is actually used on the fields. Examples of the additives for agricultural chemicals include diluents (e.g., solvent, extender, filler, and carrier), surfactants (e.g., emusifying agent, and dispersing agent), stabilizers, and binders.

The present invention is further described by the following examples.

### Synthetsis Example 1

### 1-[3-(5-Methyl-2-phenylhexylamino)propyl]piperidine

i) A mixture of 2.47 g of 5-methyl-2-phenylhexanoic acid and 1.43 g of thionyl chloride was stirred at room temperature for 27 hours, and subsequently excessive thionyl chloride was distilled off under reduced pressure at temperatures of lower than 40°C. The residue was dissolved in 5 mℓ of benzene. To the benzene solution was dropwise added under chilling with ice and vigorous stirring over a period of 15 min. a mixture of 50 mℓ of 1N aqueous sodium hydroxide and a solution of 1.42 g or 1-(3-aminopropyl)piperidine in 50 mℓ of chloroform. The stirring was continued for 30 min. under chilling with ice and for 40 min. at room temperature. The organic layer was separated and washed successively with water and saturated aqueous sodium chloride. The organic layer was then dried over anhydrous sodium sulfate and evaporated under reduced pressure to remove the solvent. There was obtained 3.0 g of 5-methyl-2-phenyl-N-(3-piperidinopropyl)hexanamide as a viscous oil, yield: 90.9%.
ii) To a solution of 3.0 g of the above-obtained protuct in 80 mℓ of ether was added 0.69 g of aluminum lithium hydride, and the resulting mixture was heated under reflux for 20 hours. To the reaction mixture was dropwise added under chilling with ice a saturated aqueous sodium sulfate solution to decompose in excessive portion of the aluminum lithium hydride. The insolubles were then removed by decantation. The organic layer was dried over anhydrous sodium sulfate and evaporated under reduced pressure to remove the solvent, yielding an oily product. The oily product was purified by silica gel column chromatography (eluent: chloroform-methanol) to give 2.6 g of the desired compound.

In a solution of the above-obtained free base in ethanol was dissolved fumaric acid of two-equivalent amount. The resulting solution was concentrated under reduced pressure to dryness, and the residue was recrystallized from ethanol to give a difumaric acid salt of the desired compound.
m.p.: 189-192°C (decompn.)

### Synthesis Example 2

### 1-[3-(1-Pentylhexylamino)propyl]piperidine

i) To a solution of 5.11 g of 6-undecanone in 20 mℓ of ethanol were successively added a solution of 3.47 g of hydroxylamine hydrochloride in 6 mℓ of water and a solution of 4.77 g of potassium hydroxide in 6 mℓ of water. The resulting mixture was heated under reflux for 3 hours. The reaction mixture was then poured into 150 mℓ of ice-containing water. The resulting aqueous mixture was made acidic by addition of 2N hydrochloric acid, and extracted with benzene. The organic layer was washed with saturated aqueous sodium chloride and evaporated under reduced pressure to remove the solvent. There was obtained 5.57 g (corresponding to theoretical amount) of 6-undecanone oxime as a pale yellow solid,
ii) To a solution of 1.85 g of the above-obtained product in 140 mℓ of ethanol was added 140 mℓ of 2N aqueous sodium hydroxide and further added 10.7 g of Raney nickel at once. The resulting mixture was stirred for 1 hour, filtered, and washed successively with water and ethanol. The filtrate and the washings were combined, diluted with water, and then extracted with chloroform. The organic layer was washed with saturated aqueous sodium chloride and evaporated under reduced pressure to remove the solvent. There was obtained a pale yellow oil. This oil was purified by silica gel column chromatography (eluent; chloroform-methanol) to give 1.00 g of 6-undecanamine as a colorless oil, yield: 58.5%.
iii) A mixture of 0.85 g of the above-obtained product and 0.80 g of 1-(3-chloropropyl)piperidine was heated in a nitrogen atmosphere at 110-120°C for 3 hours. The reaction mixture was cooled and dissolved in ethanol. To the resulting solution was added 0.41 mℓ of conc. hydrochloric acid. The mixture was stirred, and then allowed to stand after addition of ethyl acetate. Thus precipitated crystals were collected by filtration, washed with ethyl acetate, and dried to give 640 mg of a crude crystalline product. The product was recrystallized from ethanol-ethyl acetate to give 540 mg of dihydrochloride of the desired compound as a white crystalline product, yield 29.5%.
   m.p.: 233-235°C

### Synthesis Example 3

### 1-[3-[4-Methyl-1-(3-methylbutyl)pentylamino]propyl]-piperidine

i) To a solution of 5.11 g of 2,8-dimethylnonan-5-one (b.p.: 103-105°C/19 mmHg, prepared by oxidizing 2,8-dimethylnonan-5-ol with a bleaching powder; 2,8-dimethylnonan-5-ol was prepared by reaction of ethyl formate and isoamylmagnesium bromide) in 20 mℓ of ethanol were successively added an aqueous solution of 3.47 of hydroxylamine hydrochloride in 6 mℓ of water and a solution of 4.77 g of potassium hydroxide in 6 mℓ of water. The mixture was heated under reflux. The reaction mixture was then poured into 150 mℓ of ice-containing water. The aqueous mixture was made acidic by addition of 2N hydrochloric acid and extracted with benzene. The organic layer was washed with saturated aqueous sodium chloride, dried over anhydrous sodium sulfate, and evaporated under reduced pressure to remove the solvent. There was obtained 5.21 g of 2,8-dimethylnonan-5-one oxime as a pale brown oil, yield: 93.7%.
ii) To a solution of 2.78 g of the above-obtained product in 60 mℓ of ethanol was added 60 mℓ of 2N aqueous sodium hydroxide and further added 4.32 g of Raney alloy at once. The resulting mixture was stirred for 3 hour, filtered, and washed successively with ethanol and water. The filtrate and the washings were combined, diluted with water, and then extracted with chloroform. The extract was dried over anhydrous sodium sulfate and evaporated under reduced pressure to remove the solvent. There was obtained a crude product containing 2,8-dimethylnonan-5-amine. This was then isolated in the form of an oxalate. The oxalate was treated with aqueous sodium hydroxide to give a free base.
iii) A mixture of 1.55 g of the above-obtained product and 1-(3-chloropropyl)piperidine was heated to 120°C for 3.5 hours in a nitrogen atmosphere. The reaction mixture was cooled and dissolved in 10 mℓ of ethanol. To this solution were added 0.75 mℓ of conc. hydrochloric acid and ethyl acetate of a volume to make the total volume to 100 mℓ. The precipitated crystals were collected by filtration. The crystals were treated with aqueous sodium hydroxide and extracted with chloroform to obtain an oil. The oil was purified by silica gel column chromatography (eluent: chloroform-methanol) to give the desired compound as an oil.

To an ethanol solution containing the above-obtained free base was added a slightly excessive amount of 6N hydrochloric acid-ethanol. The mixture was concentrated to dryness. The residue was recrystallized from ethanol -ethyl acetate to give dihydrochloride of the desired compound as a white crystalline product.
m.p.: 249-250°C (decompn.)

### Synthesis Examples 4-25

Essentially according to the processes of the above synthesis examples, the following compounds were prepared.

### Syn Ex. No.

- 4: 1-[3-(1-Benzyl-4-methylpentylamino)propyl]piperidine, m.p. 228-231°C (decompn., as dihydrochloride)
- 5: 1-[3-(4-Methyl-1-phenylpentylamino)propyl]piperidine, m.p. 211-215°C (decompn., as dihydrochloride)
- 6: 1-[3-(3-Isopropoxy-1-phenylpropylamino)propyl]piperidine, m.p. 199-201°C (decompn., as dihydrochloride)
- 7: 1-[3-[2⁻(3-Methylbutyloxy-2-phenylethylamino)propyl]piperidine, m.p. 198-199°C (as dihydrochloride)
- 8: 1-[3-(2-Phenoxy-2-phenylethylamino)propyl]piperidine, m.p. 243-244°C (decompn., as dihydrochloride)
- 9: 3-Phenyl-2-(3-piperidinopropylamino)propyl 3-methylbutanoate, m.p. 189-190°C (decompn., as dihydrochloride)
- 10: 1-[3-[4-Methyl-1-(2-phenylethyl)pentylamino]propyl]piperidine, m.p. 220-223°C (decompn., as dihydrochloride)
- 11: 1-[3-(3-Benzyl-6-methylheptylamino)propyl]piperidine, m.p. 229-233°C (decompn., as dihydrochloride)
- 12: 1-[3-(2-Benzyl-5-methylhexylamino)propyl]piperidino, m.p. 228-230°C (decompn., as dihydrochloride)
- 13: 1-[3-[4-Methyl-1-(3-phenylpropyl)pentylamino]propyl]piperidine, m.p. 181-181.5°C (decompn., as dihydrochloride)
- 14: 1-[3-[2,3-Dimethyl-1-(3-methylbutyl)pentylamino]propyl]piperidine, m.p. 179-181°C (decompn., as difumarate)
- 15: 1-[3-[1-(1-Ethylpropyl)-4-methylpentylamino]propyl]piperidine, m.p. 210-212°C (decompn., as dihydrochloride)
- 16: 1-[3-(6-Methyl-3-phenylheptylamino)propyl]piperidine, m.p. 173-176°C (decompn., as difumarate)
- 17: 1-[3-[4,4-Dimethyl-1-(3,3-dimethylbutyl)pentylamino]propyl]piperidine, m.p. 282-284°C (decompn., as dihydrochloride)
- 18: 1-[3-[1-(3-Methylbutyl)hexylamino]propyl]piperidine, m.p. 233-236°C (decompn., as dihydrochloride)
- 19: 1-[3-[4,4-Dimethyl-1-(3-methylbutyl]pentylamino]propyl]piperidine, m.p. 256-263°C (decompn., as dihydrochloride)
- 20: 1-[3-[4-methyl-1-(3-methylbutyl)pentylamino]propyl]pyrrolidine, m.p. 248-250°C (decompn., as dihydrochloride)
- 21: 1-[3-[4-Methyl-1-(3-methylbutyl)pentylamino]propyl]perhydroazepine, m.p. 220-226°C (decompn., as dihydrochloride)
- 22: 1-[3-[4-Methyl-1-(3-methylbutyl)pentylamino]propyl]perhydroazocine, m.p. 196-198°C (decompn., as dihydrochloride)
- 23: 1-[2-[4-Methyl-1-(3-methylbutyl)pentylamino]-ethyl] piperidine, m.p. 261-263°C (decompn., as dihydrochloride)
- 24: 1-[4-(4-Methyl-1-(3-methylbutyl)pentylamino]-butyl] piperidine, m.p. 263-266°C (decompn., as dihydrochloride)
- 25: 1-[5-[4-Methyl-1-(3-methylbutyl)pentylamino]-pentyl] piperidine, m.p. 225-227°C (decompn., as dihydrochloride)

### Synthesis Example 26

### 1-[3-[N,N-Bis-(3-methylbutyl)amino]propyl]piperidine

i) A mixture of 23.2 ml of isoamylamine and 16.17g of 1-(3-chloropropyl) piperidine was heated to 120°C for 2 hours. The reaction mixture was then dissolved in 100 ml of ethanol. To this ethanol solution was added 17 ml of conc. hydrochloric acid. The mixture was allowed to stand overnight at room temperature. The precipitated crystals were collected, washed with ethanol, and dried to give 14.70 g of 1-[3(3-methylbutylamino)propyl] piperidine dihydrochloride as a white crystalline product.
   The mother liquor was concentrated to dryness, and the residue was recrystallized from 50 ml of ethanol to further obtain 3.64 g of the white crystalline product, yield 64%. m.p.: 263-265°C (decompn.)
ii) A mixture of 1.43 g of the above-obtained product, 20 ml of chloroform, and 24 ml of aqueous sodium hydroxide was stirred at room temperature. When the reaction mixture turned transparent, the mixture was chilled with ice. To the chilled mixture was dropwise added 1.21 g of isovaleroyl chloride. The mixture was then stirred for 30 min. under chilling with ice and for 1 hour at room temperature. The organic layer was separated and washed successively with 2N aqueous sodium hydroxide and saturated aqueous sodium chloride. The washed product was then dried over anhydrous sodium sulfate and evaporated to remove the solvent. There was obtained 1.46 g of N-(3-methylbutyl)-N-(3-piperidinopropyl)-3-methylbutanamide as crude crystals.
iii) In 10mℓ of tetrahydrofuran was suspended 0.76 g of lithium aluminum hydride, and this suspension was chilled with ice. To this suspension was dropwise added a solution of 1.46 g of the above-obtained product in 20 mℓ of tetrahydrofuran. The reaction mixture was heated under reflux for one hour, and then chilled with ice. To the chilled mixture were added successively ethyl acetate and saturated aqueous ammonium chloride to decompose an excessive portion of lithium aluminum hydride. The organic layer was recovered by decantation. The organic layer was washed with saturated aqueous sodium sulfate, dried over anhydrous sodium sulfate and evaporated under reduced pressure to remove the solvent. The residue was purified by silica gel column chromatography (eluent: chloroform-methanol) to give 0.81 g of the desired compound as a pale yellow oil, yield: 57%.
   To an ethanol solution containing 0.81 g of the above-obtained product was added an excessive amount of hydrochloric acid-ethanol, and the mixture was concentrated under reduced pressure to dryness. The residue was recrystallized from acetone to give 0.78 g of dihydrochloride of the desired compound as a white crystalline product, yield: 76%.
   m.p.: 182-184°C (decompn.)

### Synthesis Example 27

### 1-[3-[N-(3-Methylbutyl)-N-(4-methylpentyl)amino] propyl]piperidine

i) In the similar manner to the manner as in Synthesis Example 26, 1.09 g of 4-methyl-N-(3-methylbutyl)-N-(3-piperidinopropyl)pentanamide was prepared from 1.00 g of 1-[3-(3-methylbutylamino)propyl]piperidine dihydrochloride and 0.95 g of 4-methylpentanoyl chloride.
ii) In 10 mℓ of dry ether was suspended 0.53 g of lithium aluminum hydride. The resulting suspension was dropwise added to a solution of 1.09 g of the above-obtained product in 15 mℓ of ether. After the addition was complete, the mixture was stirred at room temperature for one hour and again chilled with ice. To the chilled mixture was added ethyl acetate to decompose an excessive portion of lithium aluminum hydride. To the mixture was then added a saturated aqueous sodium sulfate, and the organic layer was separated by decantation. The organic layer was dried over anhydrous sodium sulfate, evaporated under reduced pressure to remove the solvent. The residue was purified by silica gel column chromatography (eluent: chloroform-methanol) to give 600 mg of the desired compound as a colorless oil, yield 64%.
   To the above free base in ethanol was added two-equivalent amount (0.52 g) of fumaric acid. The mixture was heated to make a solution. To the mixture was then added ethyl acetate, and it was allowed to stand overnight. The precipitated crystals were collected by filtration, washed successively with ethyl acetate and hexane, and dried to give 0.95 g of difumarate of the desired compound as a white crystalline product, yield: 51%.
   m.p. : 138 - 140°C

### Synthesis Examples 28-42

Essentially according to the processes of the above synthesis examples, the following compounds were prepared.

### Syn Ex. No.

- 28: 1-[3-[3-Methyl-N-(2-phenoxyethyl)butylamino]propyl]piperidine, m.p 117-119°C (decompn., as dihydrochloride)
- 29: 1-[3-[3-Methyl-N-(2-phenylethyl)butylamino]propyl]piperidine, m.p. 144-146°C (decompn., as difumarate)
- 30: 1-[3-[3-Methyl-N-(3-phenylpropyl)butylamino]propyl]piperidine, m.p. 117-119°C (decompn., as difumarate)
- 31: 1-[3-[3-Methyl-N-(4-phenylbutyl)butylamino]propyl]piperidine, m.p. 112-114°C (decompn., as difumarate)
- 32: 1-[3-[N-(3-Methylbutyl)-N-(5-methylhexyl)amino]propyl]piperidine, m.p. 139-141°C (decompn., as difumarate)
- 33: 1-[3-[N-hexyl-N-(3-methylbutyl)amino]propyl]piperidine, m.p. 138-139°C (as difumarate)
- 34: 1-[3-[N-heptyl-N-(3-methylbutyl)amino]propyl]piperidine, m.p. 132-134°C (decompn. as difumarate)
- 35: 1-[3-[N-(3-Methylbutyl)-N-octyl)amino]propyl]piperidine, m.p. 127-131°C (decompn., as difumarate)
- 36: 1-[3-[N-(3-Methylbutyl)-N-nonyl)amino]propyl]piperidine, m.p. 136-138°C (decompn., as difumarate)
- 37: 1-[3-[N-(3,3-Dimethylbutyl)-N-(3-methylbutyl)amino]propyl]piperidine, m.p. 152-154°C (decompn., as difumarate)
- 38: 1-[4-[N-(3-Methylbutyl)-N-(5-methylhexyl)amino]butyl]piperidine, m.p. 130°C (as difumarate)
- 39: 1-[2-[N-(3-Methylbutyl)-N-(5-methylhexyl)amino]ethyl]piperidine, m.p. 140-141°C (decompn., as difumarate)
- 40: 1-[2-[N-(3-Methylbutyl)-N-(5-methylhexyl)amino]propyl]pyrrolidine, m.p. 134-135°C (as difumarate)
- 41: 1-[3-[N-(3-Methylbutyl)-N-(5-methylhexyl)amino]propyl]perhydroazepin, m.p. 128.5-131.5°C (as difumarate)
- 42: 1-[3-[N,N-Bis(2,3-dimethybutyl)amino]propyl]piperidine, m.p. 159-163°C (decompn., as difumarate)

### Reference Examples

### Glutamate Blocking Effect on Neuromuscular Junction of Crayfish

(1) Evaluation of glutamate blocking effect was performed according to the methods of Ishida et al [J. Physiol., 298, 301-319(1980)], and Shinozaki et al[Comp. Biochem. Physiol, 70c, 49-58(1981)]. In more detail, the opener muscle of the first walking leg of the crayfish is employed for the examination described below.
A neuromusculor sample is fixed in a vessel, and an aqueous salt solution for crayfish [composition: NaCℓ (195 mM), CaCℓ₂ (18mM), KCℓ (5.4 mM), Tris-maleate buffer (pH 7.5, 10 mM), and glucose (11 mM)] was circulated at a constant rate into the vessel.
A glass microelectrode filled with 3M KCℓ solution was inserted into the middle portion of the muscle fibre and the change of potential induced by L-glutamate (1 x 10⁻⁴M) was measured intracellularly.
The glutamate blocking effect of the compound to be tested was evaluated by determining an inhibition ratio of glutamate potential protreated by the test solution (2 x 10⁻⁵ M) for 5 min.
The results are set forth in Table 1.

**Table 1**

| Compound Tested | Glutamate Blocking Effect |
|---|---|
| Synthesis Example 1 | 99 % |
| Synthesis Example 2 | 80 % |
| Synthesis Example 3 | 99 % |
| Synthesis Example 4 | 99 % |
| Synthesis Examples 5-6 | 94 % |
| Synthesis Example 7 | 96 % |
| Synthesis Example 8 | 94 % |
| Synthesis Example 9 | 90 % |
| Synthesis Example 10 | 99 % |
| Synthesis Example 11 | 96 % |
| Synthesis Example 12 | 98 % |
| Synthesis Example 13 | 96 % |
| Synthesis Example 15 | 94 % |
| Synthesis Example 18 | 97 % |
| Synthesis Example 19 | 88 % |
| Synthesis Example 20 | 96 % |
| Synthesis Examples 21-25 | 100 % |
| Synthesis Example 26 | 91 % |
| Synthesis Example 28 | 72 % |
| Synthesis Example 29 | 80 % |
| Synthesis Example 30 | 96 % |
| Synthesis Example 33 | 83 % |
| Synthesis Example 34 | 91 % |
| Synthesis Examples 35-36 | 94 % |
| Synthesis Example 38 | 96 % |
| Synthesis Example 40 | 94 % |
| Synthesis Example 41 | 100 % |

(2) The glutamate blocking effects of the compound of the present invention as described in Synthesis Example 3 (i.e. 1-[3-[4-methyl-1-(3-methylbutyl)penty]amino]propyl]piperidine) and the known 5-methyl-1-phenyl-2-(3-piperidinopropylamino)hexan-1-ol were evaluated in the same manner as above except that the concentration of the liquid for the pretreatment was reduced from 2 x 10⁻⁵ M to 5 x 10⁻⁷ M. The results are set forth in Table 2.

**Table 2**

| Compound Tested | Glutamate Blocking Effect |
|---|---|
| 1-[3-[4-Methyl-1-(3-methylbutyl)pentylamino]propyl]piperidine (according to the invention) | 25% |
| 5-Methyl-1-phenyl-2-(3-piperidinopropylamino)hexan-1-ol (EP-A-177 245) | 6% |

(3) The neuromuscular sample which was fixed in the vessel and treated in the above manner was subsequently washed with the salt solution flowing in the vessel under circulation for 40 min. After such washing was complete, the glutamate response was measured on the wahsed neuromuscular sample.
It was confirmed that the sample treated with 5-methyl-1-phenyl-2-(3-piperidinopropylamino)hexan-1-ol showed a glutamate response at almost the same level as shown by the sample prior to the treatment. Accordingly, it was confirmed that the glutamate blocking action of the aninoalcohol derivative was not kept for sufficient period of time. In contrast, 1-[3-[4-methyl-1-(3-methylbutyl)pentylamino]propyl]piperidine of the invention showed almost no glutamate response even after it was well washed with the salt solution. The prolongation of the effect of glutamate response shown in using this alkylenediamine compound was also observed on the alkylenediamine derivatives tested in the (1) above,
(4) Acute toxicity (LD₅₀, i.v.) was measured on the alkylenediamine drivatives of the invention and the known aminoalcohol derivative. The results are set forth in Table 3.

**Table 3**

| Compound Tested | Acute Toxicity (LD₅₀) |
|---|---|
| 1-[3-(1-Pentylhexylamino] propyl]piperidine | 23.3 mg/kg |
| 1-[3-[4-Methyl-1-(3-methylbutyl)pentylamino]propyl]piperidine | 29.5 mg/kg |
| 1-[3-[N,N-Bis(3-methylbutyl)amino] propyl]piperidine | 24.8 mg/kg |
| 5-Methyl-1-phenyl-2-(3-piperidinopropylamino)hexan-1-ol | 13.6 mg/kg |

It is apparent that the alkylenediamine derivatives of the invention show less accute toxicity than the known aminoalcohol derivative.

## Claims (Claims for the following Contracting State(s): AT, CH, DE, FR, GB, IT, LI, NL, SE)

1. An alkylenediamine derivative having either formula (I) or (II): 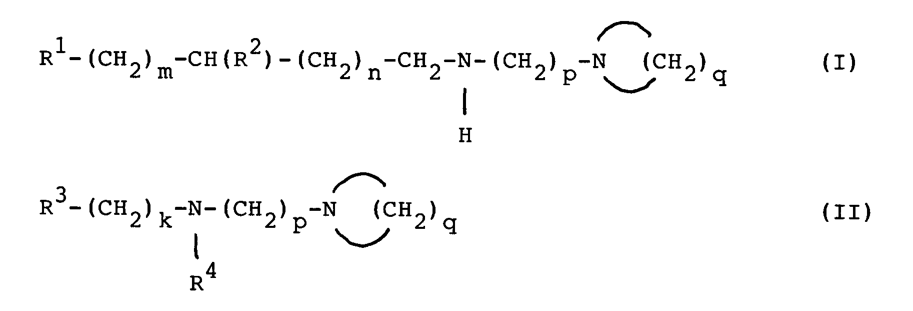 wherein
R¹ is a straight or branched chain alkyl group containing 3-8 carbon atoms, an alicyclic group containing 5-8 carbon atoms, a phenyl group, or an aralkyl group in which the alkyl moiety contains 1-4 carbon atoms;
R² is a straight or branched chain alkyl group containing 4-8 carbon atoms, an alkoxy group containing 3-11 carbon atoms, an aliphatic hydrocarbon group containing an ester bonding and 3-11 carbon atoms, an aliphatic hydrocarbon group containing an ether bonding and 3-11 carbon atoms, or an aryloxy group; and each of m and n is an integer of 0 to 2, provided that m + n does not exceed 3;
R³ is a straight or branched chain aliphatic hydrocarbon group containing 3-8 carbon atoms, an alicyclic group containing 5-8 carbon atoms, an aryl group, or an aralkyl group in which the alkyl moiety contains 1-4 carbon atoms; and k is an integer of 1 to 4;
R⁴ is a straight or branched chain aliphatic hydrocarbon group containing 3-11 carbon atoms, an aliphatic hydrocarbon group containing an ester bonding and 3-11 carbon atoms, an aliphatic hydrocarbon group containing an ether bonding and 3-11 carbon atoms, or an aralkyl group in which the alkyl moiety contains an ether bond and 2-5 carbon atoms;
p is an integer of 2 to 6;
and
q is an integer of 4 to 6.

2. An alkylenediamine derivative as claimed in claim 1, having the formula (I) in which R² is a straight or branched chain alkyl group having 4-8 carbon atoms.

3. An alkylenediamine derivative as claimed in claim 1, having the formula (II) in which R⁴ is a straight or branched chain alkyl group having 3-11 carbon atoms.

4. An alkylenediamine derivative selected from the following compounds:
1-[3-[4-methyl-1-(3-methylbutyl)pentylamino)propyl]piperidine;
1-[3-[4-methyl-1-(3-methylbutyl)pentylamino]propyl]pyrrolidine;
1-[3-[4-methyl-1-(3-methylbutyl)pentylamino]propyl]perhydroazepine;
1-[3-[4-methyl-1-(2-phenylethyl)pentylamino]propyl]piperidine;
1-[3-[4-methyl-1-(3-phenylpropyl)pentylamino]propyl]piperidine
1-[3-(1-benzyl-4-methylpentylamino)propyl]piperidine;
1-[3-(4-methyl-1-phenylpentylamino)propyl]piperidine, and
1-[3-(1-benzyl-4-methylpentylamino)propyl]perhydroazepine.

5. An alkylenediamine derivative selected from the following compounds:
1-[2-[4-methyl-1-(3-methylbutyl)pentylamino]ethyl]piperidine;
1-[2-(1-benzyl-4-methylpentylamino)ethyl]piperidine; and
1-[2-(1-benzyl-4-methylpentylamino)ethyl]pyrrolidine.

6. An alkylenediamine derivative selected from the following compounds:
3-phenyl-2-(3-piperidinopropylamino)propyl 3-methylbutanoate;
1-[3-(3-isopropoxy-1-phenylpropylamino)propyl]-piperidine; and
1-[3-(1-pentylhexylamino)propyl]piperidine.

7. An alkylenediamine derivative selected from the following compounds:
1-[2-[4-methyl-1-(3-methylbutyl)pentylamino]ethyl]piperidine;
1-[3-[4,4-dimethyl-1-(3-methylbutyl)pentylamino]propyl]piperidine;
1-[3-[4,4-dimethyl-1-(3,3-dimethylbutyl)pentylamino]propyl] piperidine;
1-[3-[2,3-dimethyl-1-(3-methylbutyl)pentylamino]propyl]piperidine;
1-[3-[1-(1-ethylpropyl)-4-methylpentylamino]propyl]piperidine;
1-[3-(1-benzyl-4-methylpentylamino)propyl]pyrrolidine;
1-[3-[4-methyl-1-(3-methylbutyl)pentylamino]propyl]perhydroazocine;
1-[4-[4-methyl-1-(3-methylbutyl)pentylamino]butyl]piperidine; and
1-[5-[4-methyl-1-(3-methylbutyl)pentylamino]pentyl]piperidine.

8. The alkylenediamine derivative:
1-[3-[1-(3-methylbutyl)hexylamino]propyl]piperidine.

## Claims (Claims for the following Contracting State(s): ES)

1. A process for the preparation of an alkylenediamine derivative having either formula (I) or (II): 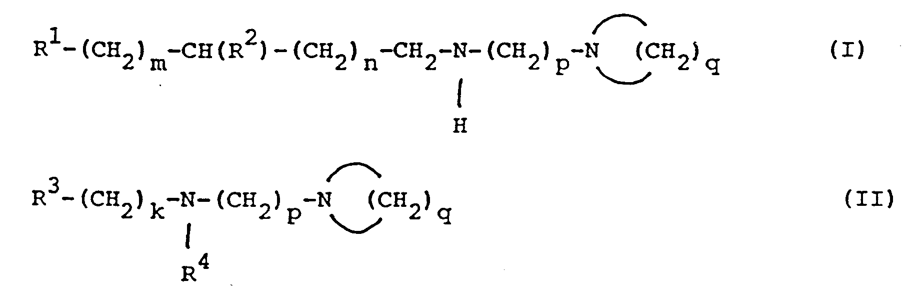 wherein
R¹ is a straight or branched chain alkyl group containing 3-8 carbon atoms, an alicyclic group containing 5-8 carbon atoms, a phenyl group, or an aralkyl group in which the alkyl moiety contains 1-4 carbon atoms;
R² is a straight or branched chain alkyl group containing 4-8 carbon atoms, an alkoxy group containing 3-11 carbon atoms, an aliphatic hydrocarbon group containing an ester bonding and 3-11 carbon atoms, an aliphatic hydrocarbon group containing an ether bonding and 3-11 carbon atoms, or an aryloxy group; and each of m and n is an integer of 0 to 2, provided that m + n does not exceed 3;
R³ is a straight or branched chain aliphatic hydrocarbon group containing 3-8 carbon atoms, an alicyclic group containing 5-8 carbon atoms, an aryl group, or an aralkyl group in which the alkyl moiety contains 1-4 carbon atoms; and k is an integer of 1 to 4;
R⁴ is a straight or branched chain aliphatic hydrocarbon group containing 3-11 carbon atoms, an aliphatic hydrocarbon group containing an ester bonding and 3-11 carbon atoms, an aliphatic hydrocarbon group containing an ether bonding and 3-11 carbon atoms, or an aralkyl group in which the alkyl moiety contains an ether bond and 2-5 carbon atoms;
p is an integer of 2 to 6 and q is an integer of 4 to 6 which comprises reacting a carboxylic acid having the formula (V):
A - COOH (V)
wherein A is R¹-(CH₂)ₘ-CH(R²)-(CH₂)ₙ- or R³(CH₂)ₖ₋₁-, or its reactive derivative with an amine derivative having the formula (VI): 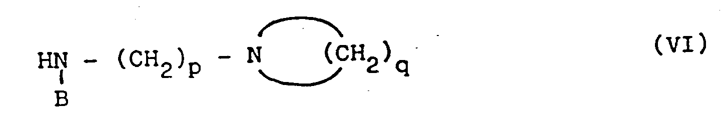 wherein B is H or R⁴, and R¹, R², R³, R⁴, k, p and q is defined above, to obtain a compound having the formula (VII): 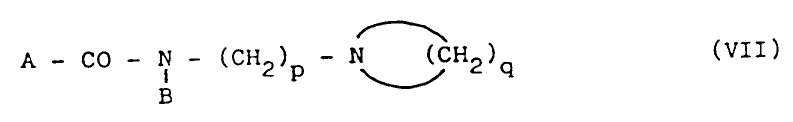 wherein each of A, B, p and q has the same meaning as above; and reducing the compound of the formula (VII).

2. A process for the preparation of an alkylenediamine derivative defined in claim 1 which comprises reacting an amine compound having the formula (VIII); 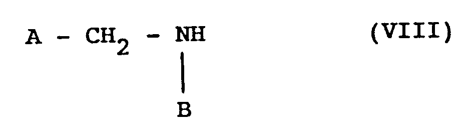 wherein A is R¹-(CH₂)ₘ-CH(R²)-(CH₂)ₙ- or R³(CH₂)ₖ₋₁- and B is H or R⁴, or its reactive derivative with a halogenated compound having the formula (IX): 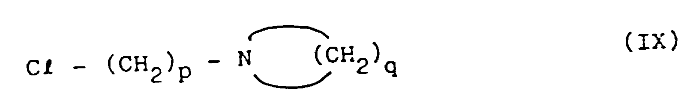 whereby R¹, R², R³, R⁴, m, n, k, p and q are defined according to claim 1

3. A process for preparing 1-[3-(5-methyl-2-phenylhexylamino)-propyl]piperidine, the process comprising reacting 5-methyl-2-phenylhexanoic acid with thionyl chloride to obtain 5-methyl-2-phenyl-N-(3-piperidinopropyl)hexanamide and reducing the obtained hexanamide.

4. A process for preparing 1-[3-(1-phenylhexylamino) propyl]-piperidine, the process reacting 6-undecanamine with 1-(3-chloropropyl)piperidine.

5. A process for preparing 1-[3-[4-methyl-1-(3-methylbutyl)-pentylamino]propyl]piperidine, the process comprising reacting 2,8-dimethylnonan-5-amine with 1-(3-chloropropyl)piperidine.

6. A process for preparing 1-[3-[N,N-bis-(3-methylbutyl)amino]-propyl]piperidine, the process comprising reacting 1-[3-(3-methylbutylamino)propyl]piperidine with isovaleroyl chloride to obtain N-(3-methyl-butyl)-N-(3-piperidinopropyl)-3-methylbutanamide, and reducing the obtained methylbutanamide.

7. A process for preparing 1-[3-[N-(3-methylbutyl)-N-(4-methyl-pentyl)amino]propyl]piperidine, the process comprising reacting 1-[3-(3-methylbutylamino)propyl] piperidine and 4-methylpentanoyl chloride to obtain 4-methyl-N-(3-methylbutyl)-N-(3-piperidinopropyl) pentanamide and reducing the obtained pentanamide.

## Patentansprüche (Patentansprüche für folgende(n) Vertragsstaat(en): AT, CH, DE, FR, GB, IT, LI, NL, SE)

1. Alkylendiamin-Derivativ mit der Formel (I) oder (II): 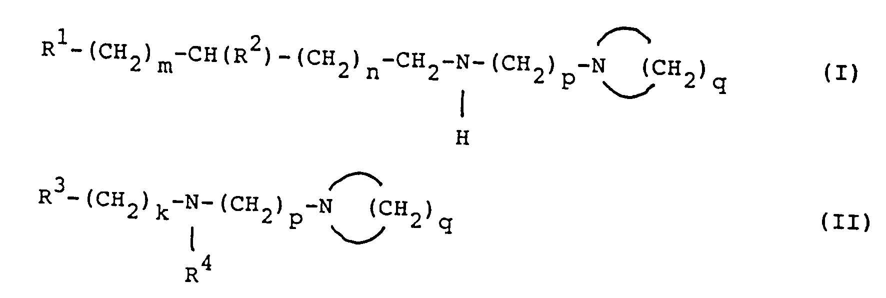 Hierin sind
R¹ eine gerade oder verzweigte Alkylgruppenkette mit 3 bis 8 Kohlenstoffatomen, eine alicyclische Gruppe mit 5 bis 8 Kohlenstoffatomen, eine Phenylgruppe oder eine Aralkylgruppe, bei der die Alkylmoietät 1 bis 4 Kohlenstoffatome enthält;
R² eine gerade oder verzweigte Alkylgruppenkette mit 4 bis 8 Kohlenstoffatomen, eine Alkoxygruppe mit 3 bis 11 Kohlenstoffatomen, eine aliphatische Kohlenwasserstoffgruppe mit einer Esterbindung und 3 bis 11 Kohlenstoffatomen, eine aliphatische Kohlenwasserstoffgruppe mit einer Ätherbindung und 3 bis 11 Kohlenstoffatomen oder eine Aryloxygruppe; sowohl m als auch n sind natürliche Zahlen zwischen 0 und 2, vorausgesetzt, daß m + n 3 nicht übersteigen;
R³ eine gerade oder verzweigte aliphatische Kohlenwasserstoffgruppenkette mit 3 bis 8 Kohlenstoffatomen, eine alicyclische Gruppe mit 5 bis 8 Kohlenstoffatomen, eine Arylgruppe oder eine Aralkylgruppe, bei der die Alkylmoietät 1 bis 4 Kohlenstoffatome enthält; k ist eine ganze Zahl zwischen 1 und 4;
R⁴ eine gerade oder verzweigte aliphatische Kohlenwasserstoffgruppenkette mit 3 bis 11 Kohlenstoffatomen, eine aliphatische Kohlenwasserstoffgruppe mit einer Esterbindung und 3 bis 11 Kohlenstoffatomen, eine aliphatische Kohlenwasserstoffgruppe mit einer Ätherbindung und 3 bis 11 Kohlenstoffatomen, oder eine Aralkylgruppe, bei der die Alkyl-Moietät eine Ätherbindung und 2 bis 5 Kohlenstoffatome enthält;
P eine natürliche Zahl zwischen 2 und 6;
und
q eine natürliche Zahl zwischen 4 und 6.

2. Alkylendiamin-Derivativ nach Anspruch 1, mit der Formel (I), wobei R² eine gerade oder verzeigte Alkylgruppenkette mit 4 bis 8 Kohlenstoffatomen ist.

3. Alkylendiamin-Derivativ nach Anspruch 1, mit der Formel (II), wobei R⁴ eine gerade oder verzweigte Alkylgruppenkette mit 3 bis 11 Kohlenstoffatomen ist.

4. Alkylendiamin-Derivativ, gewählt aus den folgenden Verbindungen:
1-[3-[4-Methyl-1-(3-Methylbutyl)Pentylamino]Propyl] Piperidin;
1-[3-[4-Methyl-1-(3-Methylbutyl)Pentylamino]Propyl] Pyrrolidin;
1-[3-[4-Methyl-1-(3-Methylbutyl)Pentylamino]Propyl] Perhydroazepin;
1-[3-[4-Methyl-1-(2-Phenylethyl)Pentylamino]Propyl] Piperidin;
1-[3-[4-Methyl-1-(3-Phenylpropyl)Pentylamino] Propyl]Piperidin;
1-[3-(1-Benzyl-4-Methylpentylamino)Propyl]Piperidin;
1-[3-(4-Methyl-1-Phenylpentylamino)Propyl]Piperidin; und
1-[3-(1-Benzyl-4-Methylpentylamino)Propyl]Perhydroazepin.

5. Alkylendiamin-Derivativ, ausgewählt aus den folgenden Verbindungen:
1-[2-[4-Methyl-1-(3-Methylbutyl)Pentylamino]Ethyl] Piperidin;
1-[2-(1- Benzyl-4-Methylpentylamino)Ethyl]Piperidin; und
1-[2-(1-Benzylin-4-Methylpentylamino)Ethyl]Pyrrolidin.

6. Alkylendiamin-Derivativ, ausgewählt aus den folgenden Verbindungen:
3-Phenyl-2-(3-Piperidinopropylamino)Propyl 3-Methylbutanat;
1-[3-(3-Isopropoxy-1-Phenylpropylamino)Propyl]Piperidin; und
1-[3-(1-Pentylhexylamino)Propyl]Piperidin.

7. Alkylendiamin-Derivativ, ausgewählt aus den folgenden Verbindungen:
1-[2-[4-Methyl-1-(3-Methylbutyl)Pentylamino]Ethyl] Piperidin;
1-[3-[4,4-Dimethyl-1-(3-Methylbutyl)Pentylamino]Propyl] Piperidin;
1-[3-[4,4-Dimethyl-1-(3,3-Dimethylbutyl)Pentylamino]Propyl] Piperidin;
1-[3-[2,3-Dimethyl-1-(3-Methylbutyl)Pentylamino]Propyl] Piperidin;
1-[3-[1-(1-Ethylpropyl)-4-Methylpentylamino]Propyl] Piperidin;_
1-[3-(1-Benzyl-4-Methylpentylamino)Propyl]Pyrrolidin;
1-[3-[4-Methyl-1-(3-Methylbutyl)Pentylamino]Propyl] Perhydroazocin;
1-[4-[4-Methyl-1-(3-Methylbutyl)Pentylamino]Butyl] Piperidin; und
1-[5-[4-Methyl-1-(3-Methylbutyl)Pentylamino]Pentyl] Piperidin.

8. Alkylendiamin-Derivativ:
1-[3-[1-(3-Methylbutyl)Hexylamino]Propyl]Piperidin.

## Patentansprüche (Patentansprüche für folgende(n) Vertragsstaat(en): ES)

1. Verfahren zur Herstellung eines Alkylendiamin-Derivativs mit der Formel (I) oder (II): 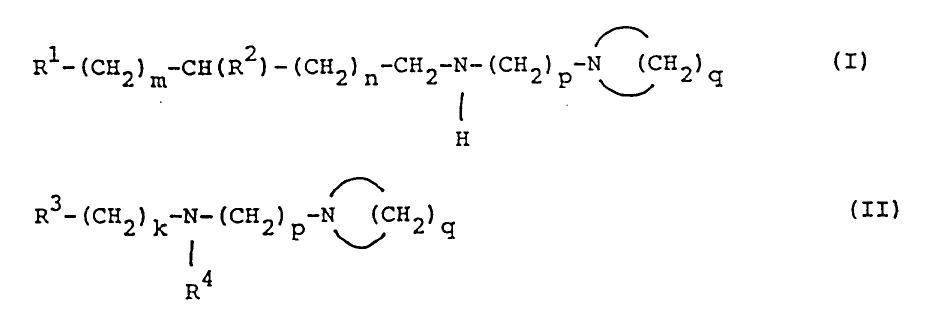 bei dem eine Carbonsäure mit der Formel (V):
A - COOH (V)
hierin bedeuten A R¹-(CH₂)ₘ-CH(R²)-(CH₂)ₙ-oder R³(CH₂)ₖ₋₁ -, oder ihr Reaktionsderivativ mit einem Amin-Derivativ mit der Formel (VI): 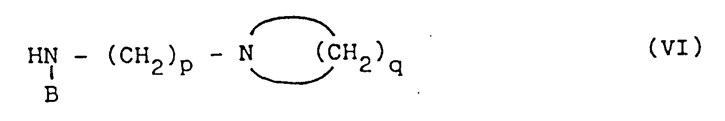 hierin bedeuten B, H oder R⁴, p eine natürliche Zahl zwischen 2 und 6 und q eine natürliche Zahl zwischen 4 und 6,
reagiert werden, um eine Verbindung mit der ormel (VII) 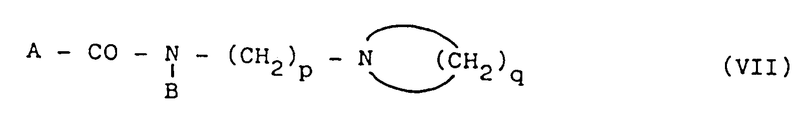 zu erhalten,
hierin bedeuten A, B, p und q dasselbe wie vorstehend;
des weiteren gehört zu dem Verfahren eine Reduzierung der Verbindung gemäß der Formel (VII).

2. Verfahren zur Herstellung eines Alkylendiamin-Derivativs mit der Formel (I) oder (II): 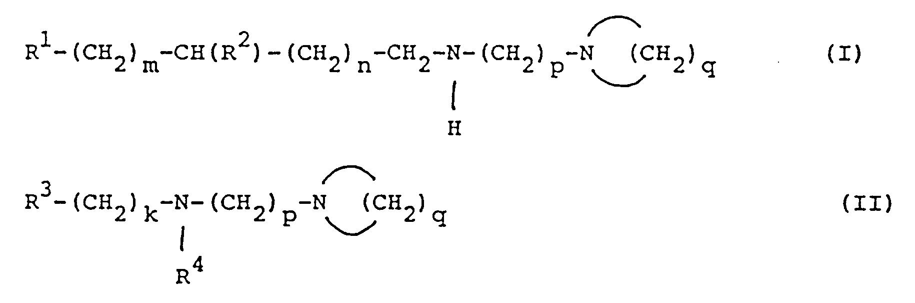 bei dem eine Aminverbindung mit der Formel (VIII) 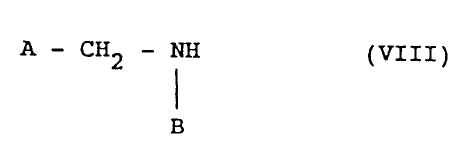 hierin bedeuten A R¹-(CH₂)ₘ-CH(R²)-(CH₂)ₙ- oder R³(CH₂)ₖ₋₁ - und B H oder R⁴, oder ein Reaktionsderivativ
mit einer halogenierten Verbindung mit der Formel (IX) 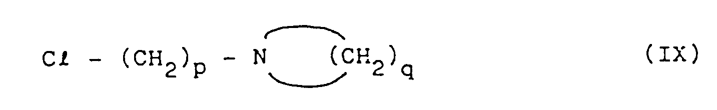 hierin bedeuten p eine natürliche Zahl zwischen 2 und 6 und q eine natürliche Zahl zwischen 4 und 6
reagiert wird.

3. Verfahren zur Herstellung von 1-[3-(5-Methyl-2-Phenylhexylamino) Propyl]Piperidin, bei dem 5-Methyl-2-Phenylhexanoicsäure mit Thionylchlorid reagiert wird, um 5-Methyl-2-Phenyl-N-(3-Piperidinopropyl)Hexanamid zu erhalten und das erhaltene Hexanamid reduziert wird.

4. Verfahren zur Herstellung von 1-[3-(1-Phenylhexylamino) Propyl] Piperidin, bei dem 6-Undecanamin mit 1-(3-Chloropropyl)Piperidin reagiert wird.

5. Verfahren zur Herstellung von 1-[3-[4-Methyl-1-(3-Methylbutyl) Pentylamino]Propyl]Piperidin, bei dem 2,8-Dimethylnonan-5-Amin mit 1-(3-Chloropropyl)Piperidin reagiert wird.

6. Verfahren zur Herstellung von 1-[3-[N,N-bis-(3-Methylbutyl)Amino] Propyl]Piperidin, bei dem 1-[3-(3-Methylbutylamino) Propyl]Piperidin mit Isovaleroylchlorid reagiert wird, um N-(3-Methyl-Butyl)-N-(3-Piperidinopropyl)-3-Methyl-Butanamid zu erhalten, und das erhaltene Methylbutanamid reduziert wird.

7. Verfahren zur Herstellung von 1-[3-[N-(3-Methylbutyl)-N-(4-Methyl-Pentyl)Amino]Propyl]Piperidin, bei dem 1-[3-(3-Methylbutylamino Propyl]Piperidin mit 4-Methylpentanoylchlorid reagiert wird, um 4-Methyl-N-(3-Methylbutyl)-N-(3-Piperidinopropyl)Pentanamid zu erhalten, und das erhaltene Pentanamid reduziert wird.

## Revendications (Revendications pour l'(les) Etat(s) contractant(s) suivant(s): AT, CH, DE, FR, GB, IT, LI, NL, SE)

1. Dérivé d'alkylènediamine ayant, soit la formule (I), soit la formule (II) : 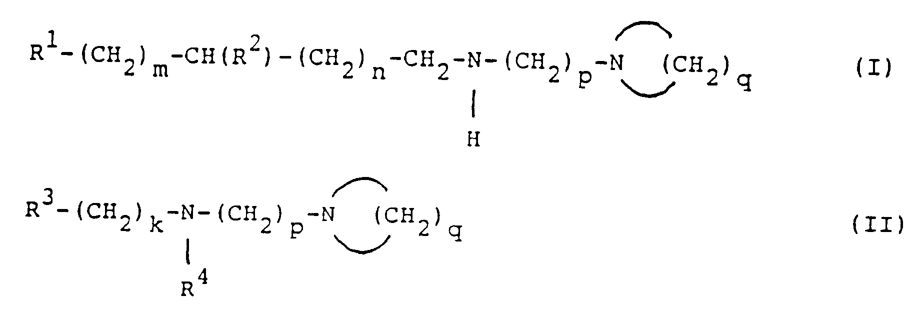 dans lesquelles :
R¹ est un groupe alkyle à chaîne droite ou ramifiée, contenant de 3 à 8 atomes de carbone, un groupe alicyclique contenant de 5 à 8 atomes de carbone, un groupe phényle, ou un groupe aralkyle dans lequel la partie alkyle contient de 1 à 4 atomes de carbone ;
R² est un groupe alkyle à chaîne droite ou ramifiée, contenant de 4 à 8 atomes de carbone, un groupe alcoxy contenant de 3 à 11 atomes de carbone, un groupe hydrocarbone aliphatique, contenant une liaison ester et de 3 à 11 atomes de carbone, un groupe hydrocarbone aliphatique contenant une liaison éther et de 3 à 11 atomes de carbone, ou un groupe aryloxy ; et chacun des m et n est un nombre entier de 0 à 2, sous la condition que m + n ne dépasse pas 3 ;
R³ est un groupe hydrocarbone aliphatique à chaîne droite ou ramifiée, contenant de 3 à 8 atomes de carbone, un groupe alicyclique contenant de 5 à 8 atomes de carbone, un groupe aryle, ou bien un groupe aralkyle dans lequel la partie alkyle contient de 1 à 4 atomes de carbone ; et k est un nombre entier de 1 à 4 ;
R⁴ est un groupe hydrocarbure aliphatique à chaîne droite ou ramifiée, contenant de 3 à 11 atomes de carbone, un groupe hydrocarbure aliphatique contenant une liaison ester et de 3 à 11 atomes de carbone, un groupe hydrocarbure aliphatique contenant une liaison éther et de 3 à 11 atomes de carbone, ou un groupe aralkyle dans lequel la partie alkyle renferme une liaison éther et de 2 à 5 atomes de carbone ;
p est un nombre entier de 2 à 6 ; et
q est un nombre entier de 4 à 6.

2. Dérivé d'alkylènediamine selon la revendication 1, ayant la formule (I) dans laquelle R² est un groupe alkyle à chaîne droite ou ramifiée ayant de 4 à 8 atomes de carbone.

3. Dérivé d'alkylènediamine selon la revendication 1, ayant la formule (II) dans laquelle R⁴ est un groupe alkyle à chaîne droite ou ramifiée, ayant de 3 à 11 atomes de carbone.

4. Dérivé d'alkylènediamine choisi parmi les composés suivants :
la 1-[3-[4-méthyl-1-(3-méthylbutyl)pentylamino]propyl]pipéridine;
la 1-[3-[4-méthyl-1-(3-méthylbutyl)pentylamino]propyl]pyrrolidine;
la 1-[3-[4-méthyl-1-(3-méthylbutyl)pentylamino]propyl]perhydroazépine;
la 1-[3-[4-méthyl-1-(2-phényléthyl)pentylamino]propyl]pipéridine;
la 1-[3-[4-méthyl-1-(3-phénylpropyl)pentylamino]propyl]pipéridine;
la 1-[3-[1-benzyl-4-méthylpentylamino]propyl]-pipéridine;
la 1-[3-(4-méthyl-1-phénylpentylamino]propyl]-pipéridine;
et
la 1-[3-(1-benzyl-4-méthylpentylamino]propyl]perhydroazépine;

5. Dérivé d'alkylènediamine choisi parmi les composés suivants :
la 1-[2-[4-méthyl-1-(3-méthylbutyl)pentylamino]éthyl]pipéridine;
la 1-[2-(1-benzyl-4-méthylpentylamino)éthyl]pipéridine; et
la 1-[2-(1-benzyl-4-méthylpentylamino)éthyl]pyrrolidine.

6. Dérivé d'alkylènediamine choisi parmi les composés suivants :
le 3-phényl-2-(3-pipéridinopropylamino)propyl 3-méthylbutanoate;
la 1-[3-(3-isopropoxy-1-phénylpropylamino)propyl]pipéridine; et
la 1-[3-(1-pentylhexylamino)propyl]pipéridine.

7. Dérivé d'alkylènediamine choisi parmi les composés suivants :
la 1-[2-[4-méthyl-1-(3-méthylbutyl)pentylamino]éthyl]pipéridine;
la 1-[3-[4,4-diméthyl-1-(3-méthylbutyl)pentylamino] propyl]pipéridine;
la 1-[3-[4,4-diméthyl-1-(3,3-diméthylbutyl)pentylamino] propyl]pipéridine;
la 1-[3-[2,3-diméthyl-1-(3-méthylbutyl)pentylamino] propyl]pipéridine;
la 1-[3-[1-(1-ethylpropyl)-4-méthylpentylamino]propyl] pipéridine;
la 1-[3-(1-benzyl-4-méthylpentylamino)propyl]pyrrolidine;
la 1-[3-[4-méthyl-1-(3-méthylbutyl)pentylamino]propyl]perhydroazocine;
la 1-[4-[4-méthyl-1-(3-méthylbutyl)pentylamino]butyl]pipéridine; et
la 1-[5-[4-méthyl-1-(3-méthylbutyl)pentylamino] pentyl]pipéridine.

8. Dérivé d'alkylènediamine :
1-[3-[1-(3-méthylbutyl)hexylamino]propyl]pipéridine.

## Revendications (Revendications pour l'(les) Etat(s) contractant(s) suivant(s): ES)

1. Procédé pour la préparation d'un dérivé d'alkylènediamine, ayant, soit la formule (I), soit la formule (II) : 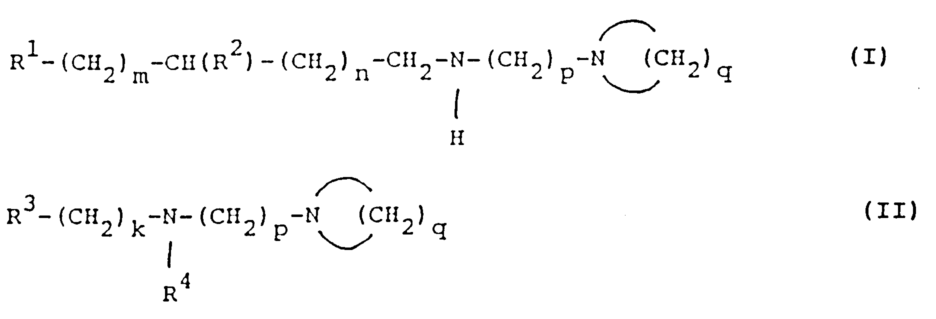 procédé selon lequel, on fait réagir un acide carboxylique ayant la formule (V) :
A - COOH (V)
dans laquelle A est R¹-(CH₂)ₘ-CH(R²)-(CH₂)ₙ- ou R³(CH₂)ₖ₋₁-, ou un de ses dérivés réactifs avec un dérivé d'amine ayant la formule (VI) : 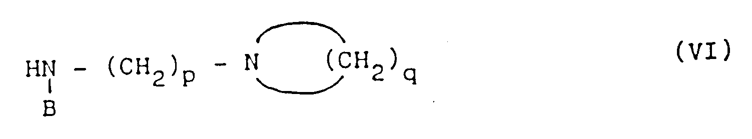 dans laquelle B est H ou R⁴, p est un nombre entier de 2 à 6 et q est un nombre entier de 4 à 6, afin d'obtenir un composé ayant la formule (VII) : 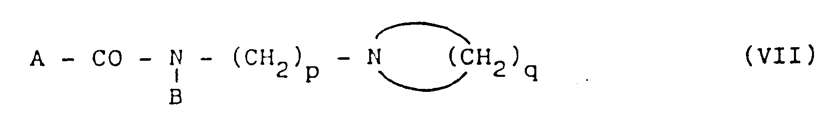 dans laquelle A, B, p et q ont chacun la même signification que ci-dessus ; et en réduisant le composé de la formule (VII).

2. Procédé pour la préparation d'un dérivé d'alkylènediamine, ayant, soit la formule (I), soit la formule (II) : 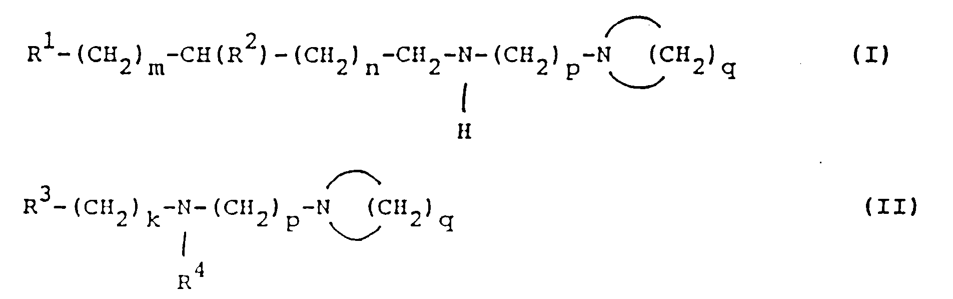 procédé selon lequel on fait réagir un composé d'amine ayant la formule (VIII) : 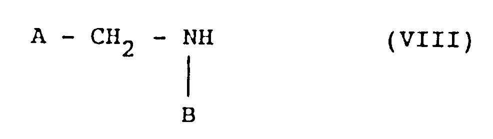 dans laquelle A est R¹-(CH₂)ₘ-CH(R²)-(CH₂)ₙ- ou R³(CH₂)ₖ₋₁-, et B est H ou R⁴, ou un de ses dérivés réactifs avec un composé halogéné ayant la formule (IX) : 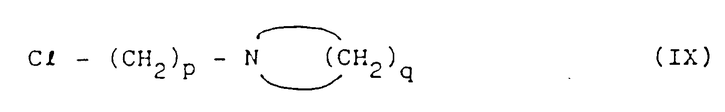 dans laquelle p est un nombre entier de 2 à 6, et q est un nombre entier de 4 à 6.

3. Procédé pour la préparation de la 1-[3-(5-méthyl-2-phénylhexylamino)-propyl]pipéridine, procédé selon lequel on fait réagir de l'acide 5-méthyl-2-phénylhexanoïque avec du chlorure de thionyle afin d'obtenir un 5-méthyl-2-phényl-N-(3-pipéridinopropyl)hexanamide, après quoi on réduit l'hexanamide obtenu.

4. Procédé pour la préparation d'une 1-[3-(1-phénylhexylamino)propyl]-pipéridine, procédé selon lequel on fait réagir une 6-undécanamine avec une 1-(3-chloropropyl)pipéridine.

5. Procédé pour la préparation d'une 1-[3-[4-méthyl-1-(3-méthylbutyl)-pentylamino]propyl]pipéridine, procédé selon lequel on fait réagir une 2,8-diméthylnonan-5-amine avec de la 1-(3-chloropropyl)pipéridine.

6. Procédé pour la préparation de la 1-[3-[N,N-bis-(3-méthylbutyl)amino]-propyl]pipéridine, procédé selon lequel on fait réagir de la 1-[3-(3-méthylbutylamino)propyl]pipéridine avec du chlorure d'isovaléroyle, afin d'obtenir le N-(3-méthyl-butyl)-N--(3-pipéridinopropyle)-3-méthylbutanamide, après quoi, on réduit le méthylbutanamide obtenu.

7. Procédé pour la préparation de la 1-[3-[N-(3-méthylbutyl)-N-(4-méthyl-pentyl)amino]propyl]pipéridine, procédé selon lequel on fait réagir la 1-[3-(3-méthylbutylamino)propyl]pipéridine et du chlorure de 4-méthylpentanoyle afin d'obtenir le 4-méthyl-N-(3-méthylbutyl)-N-(3-pipéridinopropyl)pentanamide, après quoi on réduit le pentanamide obtenu.
